(19) **Europäisches Patentamt**
**European Patent Office**
**Office européen des brevets**

(11) **EP 4 074 841 A1**

(12) **EUROPEAN PATENT APPLICATION**
published in accordance with Art. 153(4) EPC

(43) Date of publication:
**19.10.2022 Bulletin 2022/42**

(21) Application number: **20899634.8**

(22) Date of filing: **09.12.2020**

(51) International Patent Classification (IPC):
***C12Q 1/6869*** *(2018.01)*

(52) Cooperative Patent Classification (CPC):
**C12Q 1/6869**

(86) International application number:
**PCT/JP2020/045879**

(87) International publication number:
**WO 2021/117772 (17.06.2021 Gazette 2021/24)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.12.2019 JP 2019222285**

(71) Applicant: **Keio University
Tokyo, 108-8345 (JP)**

(72) Inventors:
• **KANAI, Yae
Tokyo 160-8582 (JP)**

• **ARAI, Eri
Tokyo 160-8582 (JP)**
• **KURAMOTO, Junko
Tokyo 160-8582 (JP)**
• **YOTANI, Takuya
Tokyo 103-0027 (JP)**
• **YAMADA, Yuriko
Tokyo 103-0027 (JP)**

(74) Representative: **Schiener, Jens
Wächtershäuser & Hartz
Patentanwaltspartnerschaft mbB
Weinstraße 8
80333 München (DE)**

(54) **METHOD FOR ASSESSING RISK OF DEVELOPING HEPATOCELLULAR CARCINOMA FROM NON-ALCOHOLIC STEATOHEPATITIS**

(57)    Provided is a method for assessing the risk of developing hepatocellular carcinoma from non-alcoholic steatohepatitis (NASH). A method for detecting hepatocytes or tissue comprising hepatocytes having a risk of developing hepatocellular carcinoma, the method comprising detecting DNA methylation level of a target CpG site in genomic DNA derived from hepatocytes or tissue comprising hepatocytes with NASH, and detecting hepatocytes or tissue comprising hepatocytes having a risk of developing hepatocellular carcinoma, from the detected DNA methylation level.

**(Cont. next page)**

EP 4 074 841 A1

Fig. 1

EP 4 074 841 A1

# Diagnosis of the risk of developing hepatocellular carcinoma from NASH is an unmet medical need

The first step in predicting future development of hepatocellular carcinoma is to distinguish NASH-W associated with hepatocellular carcinoma from normal liver tissue NLT

Significant difference between normal liver tissue NLT (n=36) vs NASH-W associated with hepatocellular carcinoma (n=22), and difference is sufficient
= 4,050 probes (Welch-T Test, Bonferroni correction, $\Delta\beta$>0.1) -> DNA methylation abnormalities are definitively occurring in NASH

Receiver operating characteristic (ROC) analysis to distinguish NASH-W from NLT

Area under the curve (AUC) is greater than 0.95 = a high number of probes (437) with high discriminative power

ROC plot label: 0.665 (0.909, 0.934)

Y-axis: Sensitivity (1.0, 0.8, 0.6, 0.4, 0.2, 0.0)
X-axis: Specificity (1.0, 0.8, 0.6, 0.4, 0.2, 0.0)

| Probe ID | AUC | $\Delta\beta_{NASH-NLT}$ | Probe ID | AUC | $\Delta\beta_{NASH-NLT}$ |
|---|---|---|---|---|---|
| cg18457818 | 0.994 | 0.144 | cg07959490 | 0.981 | 0.159 |
| cg19349861 | 0.992 | 0.100 | cg13161852 | 0.981 | -0.103 |
| cg07724654 | 0.990 | 0.115 | cg13468249 | 0.981 | 0.124 |
| cg14427382 | 0.990 | -0.138 | cg20251199 | 0.981 | 0.124 |
| cg21980338 | 0.990 | 0.121 | cg15284457 | 0.976 | 0.782 |
| cg24147392 | 0.990 | 0.133 | cg15646741 | 0.976 | 0.457 |
| cg03505117 | 0.987 | 0.103 | cg15920791 | 0.976 | 0.313 |
| cg15606745 | 0.987 | -0.110 | cg18159180 | 0.976 | 0.434 |
| cg16319310 | 0.987 | -0.111 | cg20326359 | 0.976 | 0.823 |
| cg03571507 | 0.986 | -0.130 | cg22216431 | 0.976 | 0.505 |
| cg12424965 | 0.986 | 0.125 | cg22793065 | 0.976 | 0.325 |
| cg14381908 | 0.986 | 0.168 | cg02073839 | 0.975 | 0.749 |
| cg25824760 | 0.986 | 0.140 | cg03468541 | 0.975 | 0.164 |
| cg20426671 | 0.985 | 0.127 | cg04449562 | 0.975 | 0.730 |
| cg14299696 | 0.984 | 0.122 | cg04867257 | 0.975 | 0.493 |
| cg12074375 | 0.984 | -0.128 | cg05707844 | 0.975 | 0.250 |
| cg21152376 | 0.984 | 0.105 | cg08941173 | 0.975 | 0.356 |
| cg25987208 | 0.983 | 0.129 | cg15936935 | 0.975 | 0.611 |
| cg07076175 | 0.982 | 0.103 | cg19105961 | 0.975 | 0.332 |
| cg07738077 | 0.982 | -0.153 | cg15470736 | 0.974 | 0.788 |
| cg07886142 | 0.982 | -0.114 | cg00112042 | 0.973 | 0.192 |
| cg11070069 | 0.982 | 0.142 | cg01593421 | 0.973 | 0.574 |
| cg11213199 | 0.982 | 0.105 | cg03000596 | 0.973 | 0.630 |
| cg12809925 | 0.982 | 0.182 | cg03022552 | 0.973 | 0.781 |
| cg22083325 | 0.982 | 0.109 | cg08912801 | 0.973 | 0.603 |
| cg07138452 | 0.981 | 0.124 | | | |
| cg07956857 | 0.981 | -0.132 | | | |

**Description**

Technical Field

The present invention relates to a method for assessing the risk of developing hepatocellular carcinoma from non-alcoholic steatohepatitis.

Background Art

Worldwide, with the rising prevalence of obesity, diabetes and the like caused by high-fat diets, the number of hepatocellular carcinoma cases with non-alcoholic steatohepatitis (NASH) as a precancerous condition is rapidly increasing and receiving much attention.

In Japan, the incidence of hepatocellular carcinoma has typically been high, with about 90% of the cases occurring in the context of chronic hepatitis/cirrhosis caused by hepatitis B or C virus infection. In recent years, however, the number of hepatocellular carcinoma cases with no apparent history of hepatitis virus infection has rapidly increased (Non Patent Literature 1), and NASH-derived hepatocellular carcinoma may account for a large proportion of these cases. Thus, the number of hepatocellular carcinoma cases with NASH as a precancerous condition is increasing worldwide. Despite this, the concept and definition of NASH is currently still in the process of being established in terms of molecular biology and histopathology.

Hepatocellular carcinoma cases occurring in the context of NASH may not be able to undergo extensive surgery or the like because their functional hepatic reserve is already reduced due to hepatitis, cirrhosis or the like at the time of diagnosis. To improve treatment outcomes, early diagnosis is crucial. However, the follow-up of the NASH-derived hepatocarcinogenic process can last for decades, and therefore strong incentives are required for patients to continue seeing their doctors. Furthermore, frequent diagnostic imaging is a significant burden to patients. Therefore, the ideal is to diagnose the carcinogenic risk of individual NASH patients and to follow up patients of the high-risk group particularly frequently. Prediction of the risk of developing hepatocellular carcinoma from NASH is considered as an unmet medical need.

By methylome analysis using a large number of tissue specimens and performed over many years, the present inventors have shown that carcinogenic factor-specific aberrant DNA methylation profiles are established in various organs from the precancerous stage (Non Patent Literature 2, etc.). In particular, they have made achievements regarding hepatocarcinogenesis since the early days of research on the epigenetics of carcinogenesis, and were the first in the world to report that DNA methylation abnormalities are already occurring in the stages of chronic hepatitis/cirrhosis caused by hepatitis virus infection (Non Patent Literature 3). Since then, they have accumulated methylome analysis of tissue specimens and have reported that the DNA methylation profile (at which CpG sites on the whole genome the cytosine base will undergo methylation modification) at the precancerous stage caused by hepatitis virus infection is inherited by hepatocellular carcinoma and determines the cancer malignancy and case prognosis (Non Patent Literatures 4, 5, etc.).

The present inventors were also among the first to analyze DNA methylation in the NASH-derived hepatocarcinogenic process. The present inventors' genome-wide DNA methylation analysis of normal liver tissue, NASH tissue, hepatocellular carcinoma tissue in the context of NASH, non-cancerous liver tissue of hepatocellular carcinoma cases in the context of hepatitis virus infection, and hepatocellular carcinoma tissue confirmed that NASH has a specific DNA methylation profile that is different from both normal liver tissue and chronic hepatitis/cirrhosis caused by hepatitis virus infection, and furthermore, revealed that the NASH-specific DNA methylation profile becomes more abnormal with the progression of NASH and is inherited by hepatocellular carcinoma occurring in the context of NASH (Non Patent Literature 6). Furthermore, the present inventors have shown that NASH-specific DNA methylation abnormalities contribute to multistage carcinogenesis through aberrant expression of several cancer-related genes (Non Patent Literature 7). Thus, the present inventors have advanced our understanding of the pathology of NASH by identifying the epigenetic changes in hepatocarcinogenesis specific to NASH.

The above studies by the present inventors show that DNA methylation abnormalities, which are stably maintained by a mechanism of maintenance methylation, are excellent biomarkers, superior to gene expression abnormalities and the like. Once they occur, DNA methylation abnormalities are inherited by the action of maintenance methyltransferase DNMT1, and thus the DNA methylation profile reflects the accumulated effects of environmental factors, including carcinogens, to which a person has been exposed over his/her lifetime. Therefore, it is possible to diagnose the carcinogenic risk by quantifying DNA methylation levels of appropriate marker CpG sites in tissues and the like that are the origin of the carcinoma. Furthermore, since DNA methylation is maintained by covalent bond on the DNA duplex, it is more stable than diagnostic indicators such as mRNA or protein expression levels, and can be detected reproducibly with a highly sensitive detection method.

Patent Literatures 1, 2 and 3 disclose methods for assessing the prognosis of renal cell carcinoma by detecting the methylation level of the CpG sites of genes such as FAM150A. Patent Literature 4 discloses a method for assessing the prognosis of endometrial carcinoma by detecting the methylation level of specific CpG sites. Patent Literature 5 discloses a method for assessing the risk of canceration of upper urinary tract urothelial tissue by detecting the methylation level of the CpG sites of genes such as TENM3. Patent Literature 6 discloses a method for evaluating the risk of hepatocellular carcinoma by detecting the methylation level of the CpG sites in the exon regions of the MGRN1 gene.
(Patent Literature 1) WO 2013/168644
(Patent Literature 2) WO 2015/129916
(Patent Literature 3) WO 2017/038983
(Patent Literature 4) WO 2020/116573
(Patent Literature 5) WO 2019/181941
(Patent Literature 6) WO 2019/039532

(Non Patent Literature 1) Int J Oncol, 43:1333-1342, 2013
(Non Patent Literature 2) Cancer sci, 101(1):36-45, 2010
(Non Patent Literature 3) Jpn J Can Res, 87(12):1210-1217, 1996
(Non Patent Literature 4) Int J Cancer, 125(12): 2854-2862, 2009
(Non Patent Literature 5) Front Genet, 5:24, 2014
(Non Patent Literature 6) Carcinogenesis, 38:261-270, 2017
(Non Patent Literature 7) J Cancer Res Clin Oncol, 146(10):2461-2477, 2020

Summary of Invention

Technical Problem

The present invention provides a method for assessing the risk of developing hepatocellular carcinoma from NASH based on the methylation level of DNA.

Solution to Problem

The present invention provides the following.
[1] A method for detecting hepatocytes or tissue comprising hepatocytes having a risk of developing hepatocellular carcinoma, the method comprising:

detecting DNA methylation level of a target CpG site in genomic DNA derived from hepatocytes or tissue comprising hepatocytes with non-alcoholic steatohepatitis and
detecting hepatocytes or tissue comprising hepatocytes having a risk of developing hepatocellular carcinoma, from the detected DNA methylation level,
wherein the target CpG site is at least one CpG site selected from the group consisting of the CpG sites located at or in the vicinity of the positions on the chromosome described in Table 1 below.

[2] A method for detecting a subject having a risk of developing hepatocellular carcinoma, the method comprising the following:

detecting DNA methylation level of a target CpG site in genomic DNA derived from hepatocytes or tissue comprising hepatocytes of a subject with non-alcoholic steatohepatitis; and
detecting a subject having a risk of developing hepatocellular carcinoma from the detected DNA methylation level,
wherein the target CpG site is at least one CpG site selected from the group consisting of the CpG sites located at or in the vicinity of the positions on the chromosome described in Table 1 below.

[3] A method for acquiring data to detect hepatocytes or tissue comprising hepatocytes having a risk of developing hepatocellular carcinoma or a subject having a risk of developing hepatocellular carcinoma, the method comprising the following:
detecting DNA methylation level of a target CpG site in genomic DNA derived from hepatocytes or tissue comprising hepatocytes of a test subject with non-alcoholic steatohepatitis; and

acquiring data on whether the hepatocytes, the tissue comprising hepatocytes, or the subject have a risk of developing hepatocellular carcinoma, from the detected DNA methylation level,
wherein the target CpG site is at least one CpG site selected from the group consisting of the CpG sites located at or in the vicinity of the positions on the chromosome described in Table 1 below.

[Table 1]

| Target CpG Site | |
| --- | --- |
| Chromosome Number | Position on the Chromosome |
| 10 | 130834003 |
| 2 | 114256392 |
| 6 | 28829182 |
| 8 | 144601781 |
| 8 | 144601800 |
| 6 | 35700382 |
| 7 | 1051703 |
| 10 | 64892616 |
| 8 | 29732714 |
| 1 | 150948024 |
| 7 | 43622659 |
| 17 | 8702099 |
| 5 | 60776693 |
| 6 | 31624387 |
| 8 | 99305661 |
| 2 | 438095 |
| 17 | 66194721 |
| 11 | 124613500 |
| 1 | 205631084 |
| 2 | 133039083 |
| 11 | 68934300 |
| 1 | 3399260 |
| 10 | 52390957 |
| 4 | 22392700 |

[4] The method according to any one of [1] to [3], wherein the target CpG site is at least one CpG site selected from the group consisting of the CpG sites at position 130,834,003 on chromosome 10, position 114,256,392 on chromosome 2, position 28,829,182 on chromosome 6, position 144,601,781 on chromosome 8, position 144,601,800 on chromosome 8, and position 35,700,382 on chromosome 6, and the CpG sites located in the vicinity thereof.
[5] The method according to any one of [1] to [4], wherein the detection of the DNA methylation level comprises detecting the DNA methylation level of the target CpG site using the genomic DNA treated with bisulfite.

Advantageous Effects of Invention

According to the present invention, the risk of developing hepatocellular carcinoma from NASH can be assessed. The present invention enables early detection of NASH patients with high risk of developing hepatocellular carcinoma, for whom follow-up is particularly required. The present invention provides incentives to seek follow-up consultations to NASH patients of the high-risk group of carcinogenesis, and contributes to the prevention of the development of hepatocellular carcinoma from NASH, or the improvement of early diagnosis and treatment outcomes of hepatocellular carcinoma.

Brief Description of Drawings

[Figure 1] Overview of receiver operating characteristic (ROC) analysis to distinguish NASH-W from NLT based on DNA methylation analysis by Infinium™ assay.
[Figure 2] Overview of the procedure for identifying candidate CpG sites for predicting carcinogenic risk from NASH.
[Figure 3] Comparison between the prediction panel of the risk of hepatocarcinogenesis and the histological findings.
[Figure 4] DNA methylation profiles of NASH specimens. Distribution of the number of marker CpGs for which the diagnostic criteria for methylation rate were met. White bar: NASH-O, black bar: NASH-W.
[Figure 5] DNA methylation rates of marker CpGs using a validation cohort. Scatter plots of DNA methylation rates at five representative marker CpGs and results of ROC analysis.
[Figure 6] Example of DNA methylation analysis by HPLC. HPLC peak patterns for a marker CpG (probe ID: cg18210511) detected in 3 NASH-W specimens and 3 NLT specimens.

Detailed Description of Invention

The present inventors have further developed their own epigenetic studies of NASH conducted so far to enable the prediction of the risk of developing hepatocellular carcinoma from NASH, an unmet medical need, based on NASH-specific DNA methylation profiles.

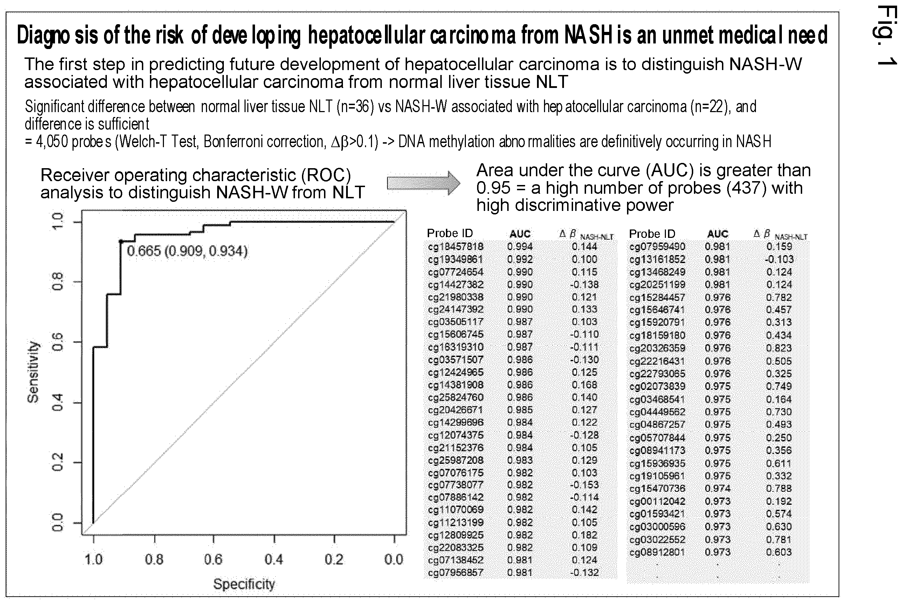

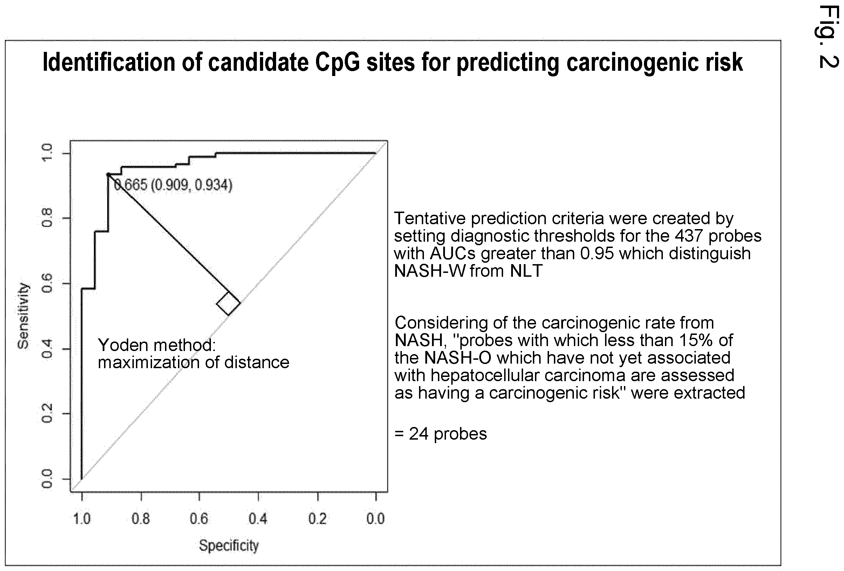

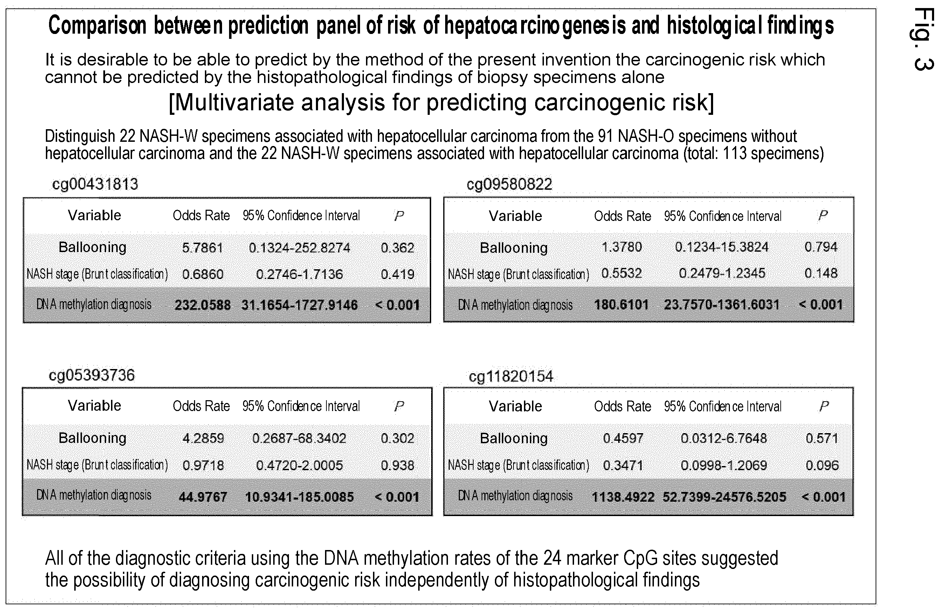

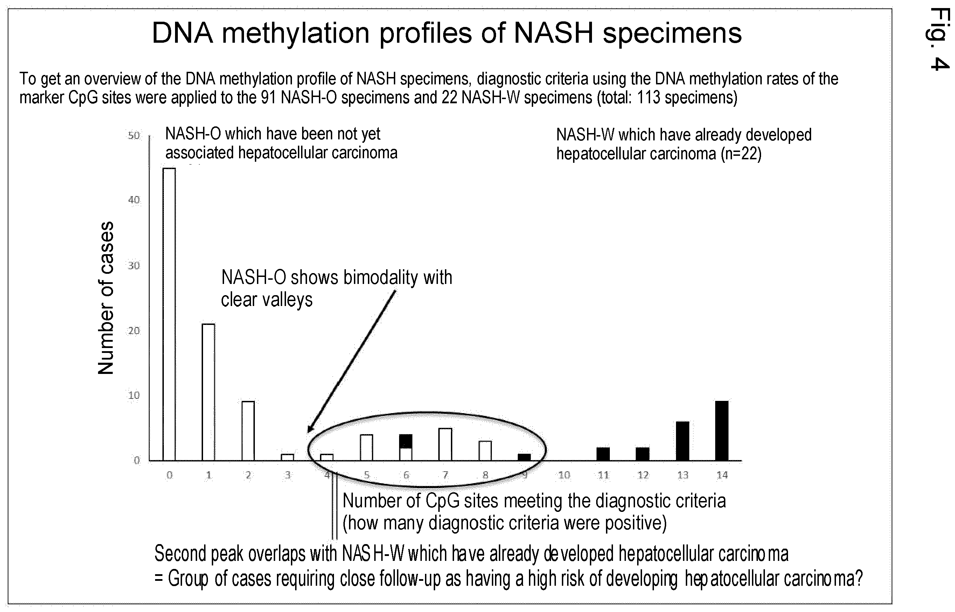

The first step in predicting future development of hepatocellular carcinoma in NASH is to distinguish liver tissue of NASH associated with hepatocellular carcinoma [NASH-W] from normal liver tissue [NLT]. As shown in the Examples described below, the present inventors first identified 437 CpG sites which discriminate NASH-W from NLT by genome-wide DNA methylation analysis (Figure 1). However, to predict the risk of hepatocarcinogenesis in actual clinical practice, it is important to be able to discriminate NASH-W associated with hepatocellular carcinoma from liver tissue of NASH without hepatocellular carcinoma [NASH-O]. On the other hand, since NASH-O may include cases in which hepatocellular carcinoma will be developed in the future, a diagnostic indicator that completely distinguishes NASH-W from NASH-O is inappropriate. Therefore, the present inventors have searched for CpG sites which can detect less than 15% of NASH-O without hepatocellular carcinoma as having carcinogenic risk among the 437 CpG sites, considering the carcinogenic rate from NASH and the like, and extracted 24 CpG sites (Figure 2). As a result of conducting a multivariate analysis on the selection of NASH-W from NASH-O and NASH-W populations, all 24 CpG sites extracted were significant in the multivariate analysis (Figure 3). The methylation status of these CpG sites in a subset of NASH-O was similar to that of NASH-W (Figure 4), which supports that the methylation level of these CpG sites reflects the risk of developing hepatocellular carcinoma. Therefore, using the DNA methylation levels of these 24 CpG sites, it is possible to predict the carcinogenic risk from NASH.

Therefore, in one embodiment, the present invention provides a method for detecting hepatocytes or tissue comprising hepatocytes having a risk of developing hepatocellular carcinoma, the method comprising the following:

detecting DNA methylation level of a target CpG site in genomic DNA derived from hepatocytes or tissue comprising hepatocytes with NASH; and
detecting hepatocytes or tissue comprising hepatocytes having a risk of developing hepatocellular carcinoma, from the detected DNA methylation level.

In another embodiment, the present invention provides a method for detecting a subject having a risk of developing hepatocellular carcinoma, the method comprising the following:

detecting DNA methylation level of a target CpG site in genomic DNA derived from hepatocytes or tissue comprising hepatocytes of a subject with NASH; and
detecting a subject having a risk of developing hepatocellular carcinoma from the detected DNA methylation level.

In another embodiment, the present invention provides a method for acquiring data to detect hepatocytes or tissue comprising hepatocytes having a risk of developing hepatocellular carcinoma or a subject having a risk of developing hepatocellular carcinoma, the method comprising the following:

detecting DNA methylation level of a target CpG site in genomic DNA derived from hepatocytes or tissue comprising hepatocytes of a subject with NASH; and
acquiring data on whether the hepatocytes, the tissue comprising hepatocytes, or the subject have a risk of developing hepatocellular carcinoma, from the detected DNA methylation level.

In the following present description, the above method for detecting hepatocytes or tissue comprising hepatocytes having a risk of developing hepatocellular carcinoma, method for detecting a subject having a risk of developing hepatocellular carcinoma, and method for acquiring data to detect hepatocytes or tissue comprising hepatocytes having a risk of developing hepatocellular carcinoma or a subject having a risk of developing hepatocellular carcinoma are also collectively referred to as "the method of the present invention".

In the present description, the "CpG site" means a site where cytosine (C) and guanine (G) are bonded by a phosphodiester bond (p) in DNA. A region where CpG sites appear with high frequency is called a CpG island.

In the present description, "DNA methylation" means a state in which the carbon at position 5 of cytosine is methylated in DNA. Moreover, in the present description, the "DNA methylation level" of a CpG site means the proportion of methylated DNA at the CpG site. In the present description, a high or low DNA methylation level means that the proportion of methylated DNA is high or low, respectively.

The " subject" to whom the method of the present invention is applied can be any subject with NASH. Preferably, the " subject" is a NASH patient in need of assessing the risk of developing hepatocellular carcinoma, and examples thereof include a NASH patient who has not yet developed hepatocellular carcinoma.

The definitive diagnosis of NASH is established by liver biopsy from patients with findings of fatty liver. The diagnosis of fatty liver can be made by abdominal ultrasonography or abdominal CT scan. More specifically, a patient is diagnosed with NASH when the patient meets the following three criteria: i) non-alcoholic (not drink excessively, e.g., an alcohol consumption of 20 g or less per day), ii) confirmation of steatohepatitis by liver biopsy findings, and iii) no confirmation of liver damage from other causes (such as hepatitis virus infection).

Examples of the target CpG site used in the method of the present invention include the CpG sites shown in Tables 2A and B below, and the CpG sites located in the vicinity thereof. In the present description, the position of the CpG site on the chromosome is expressed based on the position on the NCBI database Genome Build 37, which is a human reference genome sequence. In the present description, the "vicinity" of a position on a chromosome refers to a region from 200 bases upstream (5' side of DNA) to 200 bases downstream (3' side of DNA), preferably from 100 bases upstream to 100 bases downstream, more preferably from 50 bases upstream to 50 bases downstream, and further more preferably from 20 bases upstream to 20 bases downstream from that position on the chromosome. Alternatively, the CpG sites contained in the same CpG island as a certain CpG site are considered to be located in the vicinity of each other.

[Table 2]

| A | | |
|---|---|---|
| Chromosome Number | Position on the Chromosome (Build 37) | DNA Methylation Status In High-Risk Group |
| 2 | 114256392 | Increased |
| 8 | 144601781 | Increased |
| 8 | 144601800 | Increased |
| 7 | 1051703 | Increased |
| 10 | 64892616 | Increased |
| 8 | 29732714 | Increased |
| 1 | 150948024 | Increased |
| 7 | 43622659 | Increased |
| 17 | 8702099 | Increased |
| 5 | 60776693 | Increased |
| 6 | 31624387 | Increased |
| 17 | 66194721 | Increased |
| 11 | 124613500 | Increased |
| 1 | 205631084 | Increased |

(continued)

| A | | |
|---|---|---|
| Chromosome Number | Position on the Chromosome (Build 37) | DNA Methylation Status In High-Risk Group |
| 4 | 22392700 | Increased |
| B | | |
| Chromosome Number | Position on the Chromosome (Build 37) | DNA Methylation Status In High-Risk Group |
| 10 | 130834003 | Reduced |
| 6 | 28829182 | Reduced |
| 6 | 35700382 | Reduced |
| 8 | 99305661 | Reduced |
| 2 | 438095 | Reduced |
| 2 | 133039083 | Reduced |
| 11 | 68934300 | Reduced |
| 1 | 3399260 | Reduced |
| 10 | 52390957 | Reduced |

The target CpG site used in the method of the present invention may be at least one selected from the group consisting of the CpG sites shown in Tables 2A and B, and the CpG sites located in the vicinity thereof. For example, any one, any two or more, or any five or more selected from the group consisting of the CpG sites shown in Table 2A and the CpG sites located in the vicinity thereof, or all of them may be used as the target CpG site; any one, any two or more, or any five or more selected from the group consisting of the CpG sites shown in Table 2B and the CpG sites located in the vicinity thereof, or all of them may be used as the target CpG site; and any one, any two or more, or any five or more selected from the group consisting of the CpG sites shown in Tables 2A and B and the CpG sites located in the vicinity thereof, or all of them may be used as the target CpG site.

Preferably, any one, any two or more, or any five or more selected from the group consisting of the CpG sites shown in Tables 2A and B, or all of them may be used as the target CpG site in the method of the present invention.

More preferably, any one, any two or more, or any five or more selected from the group consisting of the CpG sites at position 130,834,003 on chromosome 10, position 114,256,392 on chromosome 2, position 28,829,182 on chromosome 6, position 144,601,781 on chromosome 8, position 144,601,800 on chromosome 8, and position 35,700,382 on chromosome 6, and the CpG sites located in the vicinity thereof, or all of them are used as the target CpG site in the method of the present invention.

Furthermore preferably, in the method of the present invention, any one, two, three, four, or five selected from the group consisting of the CpG sites at position 130,834,003 on chromosome 10, position 114,256,392 on chromosome 2, position 28,829,182 on chromosome 6, position 144,601,781 on chromosome 8, position 144,601,800 on chromosome 8, and position 35,700,382 on chromosome 6, or all six of them are used as the target CpG site.

In the method of the present invention, the DNA methylation levels of the above target CpG sites in genomic DNA derived from hepatocytes or tissue comprising hepatocytes with NASH are used as an index to assess the risk of developing hepatocellular carcinoma in the hepatocytes, the tissue comprising hepatocytes, or the subject from whom those cells or tissue are derived. Therefore, the target CpG site can be used as a marker for assessing the risk of developing hepatocellular carcinoma from NASH based on its DNA methylation level.

The genomic DNA used in the method of the present invention to detect the methylation levels can be prepared from hepatocytes or tissue comprising hepatocytes with NASH, for example, liver tissue or hepatocytes collected by surgery or biopsy on a NASH patient or biopsy for definitive NASH diagnosis. The liver tissue or hepatocytes can be fresh liver tissue collected from a living body, frozen liver tissue frozen after collection, liver tissue fixed in formalin and embedded in paraffin after collection, the hepatocytes contained in theses tissues, and the like. Among these, frozen liver tissue or hepatocytes are preferable from the viewpoint of suppressing the degradation of genomic DNA and the like and more efficiently detecting the DNA methylation level.

The method for preparing the genomic DNA from hepatocytes or tissue comprising hepatocytes is not particularly limited, and a known method can be appropriately selected and used. Examples of known methods for preparing DNA include the phenol-chloroform method, or a DNA extraction method using a commercially available DNA extraction kit, for example, QIAamp DNA Mini kit (manufactured by Qiagen), Clean Columns (manufactured by NexTec), AquaPure (manufactured by Bio-Rad), ZR Plant/Seed DNA Kit (manufactured by Zymo Research), prepGEM (manufactured by ZyGEM), and BuccalQuick (manufactured by TrimGen) and the like.

Preferably, the prepared genomic DNA is treated with bisulfite. The method for the bisulfite treatment of DNA is not particularly limited, and a known method can be appropriately selected and used. Examples of known methods for the bisulfite treatment include methods using a commercially available kit such as an EZ DNA Methylation-Gold™ Kit (manufactured by Zymo Research), EpiTect Bisulfite Kit (manufactured by Qiagen), MethylEasy (manufactured by Human Genetics Signatures Pty), Cells-to-CpG Bisulfite Conversion Kit (manufactured by Applied Biosystems), and CpGenome Turbo Bisulfite Modification Kit (manufactured by MERCK MILLIPORE). As a result of bisulfite treatment, unmethylated cytosine residues of genomic DNA are converted to uracil, but methylated cytosine residues are not converted, and remain as cytosine (see Nucleic Acids Res, 1994, 22:2990-7).

Furthermore, the bisulfite-treated DNA may be amplified. The method of amplification is not particularly limited, but PCR is preferably used. As for the method and conditions of amplification, known methods and conditions can be appropriately selected and used according to the sequence, length, amount, and the like of the DNA to be amplified.

When amplifying the bisulfite-treated DNA by PCR or the like, DNA containing at least one of the target CpG sites described above may be amplified. The chain length of the PCR amplification products can be appropriately selected while considering factors such as the shortening of the PCR amplification time, as well as the shortening of methylation level detection time and the accuracy of methylation level detection. For example, the chain length of the PCR amplification products is preferably 500 bp or less, more preferably 300 bp or less, and further more preferably 100 bp or less, while the lower limit is 30 to 40 bp, which is the chain length of the PCR amplification products when using a primer of around 15mer which can avoid non-specific hybridization in PCR. Alternatively, it is preferable to design the primer to have a rich content of the target CpG site in the PCR amplification products.

In the method of the present invention, the method for detecting the DNA methylation level of a CpG site may be a method that can quantify the DNA methylation level of a given CpG site, and a known method can be appropriately selected. Examples of such known method include single-base extension reaction using a methylation/unmethylation-specific probe, mass spectrometry, pyrosequencing (registered trademark) (Anal Biochem, 2000, 10:103-110), methylation-sensitive highresolution melting [MS-HRM] curve analysis (Nat Protoc, 2008, 3:1903-8), methylation-specific polymerase chain reaction [MS-PCR] using real-time quantitative PCR, bisulfite direct sequencing, bisulfite cloning sequencing, COBRA (analysis by the combined use of bisulfite and a restriction enzyme), and a method using ion exchange chromatography (see WO 2014/136930).

The single-base extension reaction using a methylation/unmethylation-specific probe detects the methylation of DNA at a CpG site by utilizing a single-base extension reaction using a probe constructed so as to have a base complementary to methylated cytosine or unmethylated cytosine at the 3'-terminus. Preferable examples of this method include a bead array method (for example, Infinium™ assay provided by Illumina, Inc.). The specific procedure for this technique is exemplified below.

First, the whole genome is amplified using the genomic DNA thus treated with bisulfite as a template, and enzymatic fragmentation (usually, fragmentation into about 300 to 600 bp) is performed to dissociate it into a single strand. On the other hand, a probe, which hybridizes to genomic DNA converted by bisulfite treatment, and in which the base at the 3'-terminus of the probe is a base complementary to cytosine at the target CpG site, is prepared. That is, when the CpG site is methylated, the base at the 3'-terminus of the probe is guanine, whereas when the CpG site is not methylated, the base at the 3'-terminus of the probe is adenine. These two types of probe different only in the base at the 3'-terminus complementary to the target CpG site are hybridized to the single-stranded DNA fragment, and a single-base extension reaction is performed in the presence of a fluorescently labeled base. As a result, when the CpG site of the single-stranded fragment is methylated, a fluorescently labeled base is incorporated into the probe in which the 3'-terminal base is guanine (probe for methylation detection) by a single-base extension reaction, but no fluorescently labeled base is incorporated into the probe in which the 3'-terminal base is adenine (probe for unmethylation detection) as no single-base extension reaction occurs due to the mismatch of the 3'-terminal base. On the other hand, when the CpG site of the single-stranded fragment is not methylated, a fluorescently labeled base is incorporated into the probe for unmethylation detection, but no fluorescently labeled base is incorporated into the probe for methylation detection. Therefore, the DNA methylation level of the target CpG site can be calculated from the intensity of fluorescence emitted by the probe for methylation detection and/or the probe for unmethylation detection.

Alternatively, a probe which hybridizes to genomic DNA converted by bisulfite treatment, and in which the 3'-terminal base of the probe is a base complementary to guanine at the target CpG site, may be used instead of the probe for methylation detection and the probe for unmethylation detection. Then, this probe is hybridized to the single-stranded DNA fragment, and a single-base extension reaction is performed in the presence of guanine labeled with a fluorescent substance and/or adenine labeled with a fluorescent dye different from the fluorescent substance. As a result, when the CpG site is methylated, fluorescently labeled guanine is incorporated into the probe, whereas when the CpG site is not methylated, fluorescently labeled adenine is incorporated into the probe. Therefore, the DNA methylation level of the target CpG site can be calculated from the intensity of the fluorescence emitted by each fluorescent substance incorporated into the probe.

In the detection of methylation levels by mass spectrometry, for example, bisulfite-treated genomic DNA containing a target CpG site is amplified, followed by transcription into RNA and uracil-specific cleavage by RNase to produce RNA fragments of different lengths according to DNA methylation. The obtained RNA fragments are subjected to mass spectrometry, which allows to separate and detect the methylated fragments and the unmethylated fragments according to the difference in molecular weight. The methylation level of DNA is calculated from the mass ratio between the methylated fragments and the unmethylated fragments. Examples of such mass spectrometry method include MassARRAY (registered trademark) (see Mutat Res, 2005, 573:83-95). The primer for amplifying the DNA containing the target CpG site can be designed using EpiDesigner (manufactured by SEQUENOM, primer design software for MassARRAY) and the like. For the mass spectrometry of the RNA fragments, MALDI-TOF MAS (for example, MassARRAY Analyzer 4 manufactured by SEQUENOM), which can detect the difference in mass of a single base, can be used.

In pyrosequencing, bisulfite-treated genomic DNA containing the target CpG site is amplified. By amplification, uracil in the template DNA is converted to thymine. The amplified DNA is dissociated into single strands, and extension reactions are performed on a region containing the CpG site while adding bases in order of type, and the type of base incorporated is measured based on luminescence intensity. The DNA methylation level is calculated by comparing the intensity of luminescence derived from the methylated cytosine residues (luminescence intensity of cytosine) with the intensity of luminescence derived from the unmethylated cytosine residues (luminescence intensity of thymine).

In methylation-sensitive high resolution melting curve analysis (MS-HRM), the bisulfite-treated genomic DNA containing the target CpG site is amplified in a reaction system containing an intercalator which emits fluorescence when inserted between DNA duplexes. Then, the temperature of the reaction system is changed to detect the change in the intensity of fluorescence from the intercalator. The melting curve of the DNA containing the target CpG site is compared with the melting curve of the methylated/unmethylated controls to calculate the DNA methylation level of the target CpG site.

In the methylation-specific quantitative PCR method, a primer set capable of amplifying when the target CpG site is methylated and a primer set capable of amplifying when the target CpG site is not methylated are used to amplify the bisulfite-treated genomic DNA containing the target CpG site. The DNA methylation level of the target CpG site is calculated by comparing the amounts of the amplification products obtained in each reaction, that is, the amount of methylated CpG site-specific amplification product with the amount of unmethylated CpG site-specific amplification product.

Alternatively, in the methylation-specific quantitative PCR method, oligonucleotide probes capable of hybridizing when the target CpG site is and is not methylated are each prepared. Each probe is labeled with a fluorescent reporter dye and a fluorescent quencher dye different from each other. The probe is hybridized to the bisulfite-treated genomic DNA containing the target CpG site, and then amplified, and the fluorescence emitted by the fluorescent reporter dye due to the degradation of the probe associated with amplification is detected. The DNA methylation level of the target CpG site is calculated by comparing the intensity of the fluorescence emitted by the fluorescent reporter dye specific to the methylated CpG site with the intensity of the fluorescence emitted by the fluorescent reporter dye specific to the unmethylated CpG site. Examples of such a method include a quantitative PCR method using a TaqMan (registered trademark) probe (for example, MethyLight assay).

In bisulfite direct sequencing, a direct sequencing reaction is performed using the bisulfite-treated genomic DNA containing the target CpG site as a template. Then, the DNA methylation level of the target CpG site is calculated by comparing the fluorescence intensity based on the determined base sequence, that is, the luminescence intensity derived from the methylated cytosine residues (luminescence intensity of cytosine) with the luminescence intensity derived from the unmethylated cytosine residues (luminescence intensity of thymine).

In bisulfite cloning sequencing, the bisulfite-treated genomic DNA containing the target CpG site is cloned by PCR reaction or the like, and the base sequences of the plurality of cloning products obtained are each determined. The DNA methylation level of the target CpG site is calculated by comparing the number of cloning products having a methylated CpG site-specific base sequence, with the number of cloning products having a unmethylated CpG site-specific base sequence.

In COBRA, the genomic DNA treated with bisulfite and containing the target CpG site is amplified, and next, the amplified product is treated with a restriction enzyme that recognizes the sites having a different sequence depending on whether the target CpG site is methylated or not, followed by electrophoresis. The DNA methylation level at the target CpG site is calculated by quantifying the band of the restriction enzyme fragment derived from the methylated CpG site and the restriction enzyme fragment derived from the unmethylated CpG site, which have been fractionated by electrophoresis.

In a method using ion exchange chromatography (see WO 2014/136930), first, the genomic DNA treated with bisulfite is fragmented, and fragments containing a target CpG site are amplified by PCR or the like. The chain length of the PCR amplification products can be appropriately selected while considering factors such as shortening the PCR amplification time, and shortening the analysis time and preserving the separation performance in the ion exchange chromatography. For example, the chain length of the PCR amplification products is preferably 500 bp or less, more preferably 300 bp or less, and further preferably 100 bp or less, on the other hand, the lower limit of the chain length of the PCR amplification products is 30 to 40 bp, which is the chain length of the PCR amplification products when using a primer of around 15mer which can avoid non-specific hybridization in PCR. On the other hand, it is preferable to design the primer to have a rich content of CpG site in the PCR amplification products.

Next, the amplified DNA fragments are subjected to ion exchange chromatography to separate the DNA in which the CpG sites are methylated from the DNA in which the CpG sites are not methylated. The unmethylated cytosine residues of genomic DNA are converted to uracil by bisulfite treatment and then further converted to thymine by PCR. On the other hand, the methylated cytosine residues remain as cytosine even after a bisulfite treatment and PCR. Due to this difference in base, the fragments containing methylated cytosine (methylated fragments) and the unmethylated fragments are detected as separate peaks with different retention times in ion exchange chromatography. That is, the methylated fragments are detected as peaks having a shorter retention time than the unmethylated fragments. Therefore, it is possible to determine whether the DNA at the CpG site is methylated or not based on the retention time of the peak of a detection signal in ion exchange chromatography. Furthermore, when the amplified fragment contains a plurality of CpG sites, the more CpG sites are methylated, the shorter the retention time of the peak is. Therefore, the DNA methylation level of the CpG site can be calculated based on the retention time of the peak. Alternatively, it is also possible to calculate the abundance and abundance ratio of each methylated fragment and unmethylated fragment based on the area or height of the peak.

Preferably, the DNA methylation level of the target CpG site is assessed from the peak of the detection signal of ion exchange chromatography for the amplified fragment, by comparison with a sample (control) having a known DNA methylation level of the target CpG site or by using a calibration curve prepared in advance using a sample having a known DNA methylation level. Alternatively, a retention time serving as a reference (also referred to as the reference retention time in this description) for separating the retention time of the peak of a methylated fragment having a highly methylated CpG site from the retention time of the peak of a fragment having a low methylation level using a sample having a known DNA methylation level, is determined in advance. For example, a fragment detected at a retention time earlier than the reference retention time is determined to be highly methylated DNA.

The ion exchange chromatography performed in the above method is preferably an anion exchange chromatography. The packing material of the column is not particularly limited as long as it is made of base material particles having a strong cationic group on the surface, but base material particles having both a strong cationic group and a weak cationic group on the packing material surface, as shown in WO 2012/108516, are preferable. More preferably, the base material particles are base material particles containing coated polymer particles in which a layer of a hydrophilic polymer having a strong cationic group (preferably a quaternary ammonium salt) is copolymerized on the surface of hydrophobic crosslinked polymer particles, and a weak cationic group (preferably a tertiary amino group) introduced on the surface of the coated polymer particle. The column temperature in the chromatographic analysis is preferably 30°C or more and less than 90°C.

The methods that can be suitably used as a "method for detecting the DNA methylation level" in the present invention have been exemplified above, but they are not limited thereto. In the method illustrated above, genomic DNA prepared from hepatocytes or tissue comprising hepatocytes with NASH (hereinafter also referred to as "test cells or tissue") is subjected to a bisulfite treatment. Therefore, the genomic DNA used for detecting the DNA methylation level of a CpG site in the method of the present invention is preferably bisulfite-treated genomic DNA derived from a test cell or a tissue containing the same.

In the method of the present invention, the risk of developing hepatocellular carcinoma in the test cells or tissue, or a subject from whom these are derived is assessed from the detected DNA methylation level of the target CpG site. A specific index for assessment can be appropriately set by a person skilled in the art according to the method for detecting the DNA methylation level.

An embodiment of the procedure for assessing the risk of developing hepatocellular carcinoma will be described below. In a first embodiment, first, for each DNA methylation level detection method, a receiver operating characteristic (ROC) analysis is performed for each target CpG site to obtain the sensitivity (positive rate) and specificity (negative rate), then the DNA methylation level at which the sum of the sensitivity and the specificity is maximum is set as an index (cutoff value).

In the first embodiment, for the CpG sites shown in Table 2A and the CpG sites located in the vicinity thereof, when the DNA methylation level detected in the method of the present invention is higher than the cutoff value, the DNA methylation level is considered to have exceeded the diagnostic threshold, and the test cells or tissue, or the subject can be assessed as having a risk of developing hepatocellular carcinoma. On the other hand, for the CpG sites shown in Table 2B and the CpG sites located in the vicinity thereof, when the DNA methylation level detected in the method of the present invention is lower than the cutoff value, the DNA methylation level is considered to have exceeded the diagnostic threshold, and the test cells or tissue, or the subject can be assessed as having a risk of developing hepatocellular carcinoma.

In the first embodiment, when the methylation levels of a plurality of CpG sites are detected, the number or ratio of CpG sites of which the DNA methylation level exceeds the diagnostic threshold can be used as an index for assessing the risk of developing hepatocellular carcinoma. For example, test cells or tissue, or a subject can be assessed as having a risk of developing hepatocellular carcinoma when the methylation levels of all the investigated CpG sites exceed the diagnostic threshold. Alternatively, test cells or tissue, or a subject can be assessed as having a risk of developing hepatocellular carcinoma when the methylation levels of a certain percentage or more of the investigated CpG sites exceed the diagnostic threshold. Alternatively, test cells or tissue, or a subject can be assessed as having a risk of developing hepatocellular carcinoma when the methylation levels of a certain number or more of the CpG sites exceed the diagnostic threshold.

In a second embodiment of the procedure for risk assessment, the methylation level of a CpG site is determined, or the risk of developing hepatocellular carcinoma is assessed by performing the method using ionexchange chromatography described above (see WO 2014/136930, hereafter also referred to simply as chromatography) on the DNA (sample) containing the target CpG site and comparing the retention time of the peak of the obtained detection signal with the retention time for the DNA containing the unmethylated target CpG site (negative control) or the DNA containing the methylated target CpG site (positive control).

In the second embodiment, for the CpG sites shown in Table 2A and the CpG sites located in the vicinity thereof, when a peak with a shorter retention time than that of the negative control is detected from the sample, the sample is assessed as being methylated, and the test cells or tissue, or the subject can be assessed as having a risk of developing hepatocellular carcinoma. Alternatively, when a peak with a retention time similar to that of the positive control is detected from the sample, the sample is assessed as being methylated, and the test cells or tissue, or the subject can be assessed as having a risk of developing hepatocellular carcinoma. On the other hand, for the CpG sites shown in Table 2B and the CpG sites located in the vicinity thereof, when a peak with a longer retention time than that of the positive control is detected from the sample, the sample is assessed as not being methylated, and the test cells or tissue, or the subject can be assessed as having a risk of developing hepatocellular carcinoma. Alternatively, when a peak with a retention time similar to that of the negative control is detected from the sample, the sample is assessed as not being methylated, and the test cells or tissue, or the subject can be assessed as having a risk of developing hepatocellular carcinoma.

In the first and second embodiments of the procedure for risk assessment described above, any of the CpG sites shown in Tables 2A and B above and the CpG sites located in the vicinity thereof may be used as the target CpG site used for detecting DNA methylation level. Of these, when used in the second embodiment or the chromatography described above, the target CpG site preferably has the following characteristics: 1) presence of a relatively high number of CpG sites in the vicinity; 2) high degree of separation between the peaks of the methylated and unmethylated controls in ion exchange chromatography analysis; or 3) strong association between methylation level and development of hepatocellular carcinoma. Based on these, the preferable target CpG sites for the second embodiment or the chromatography described above include the six CpG sites at position 130,834,003 on chromosome 10, position 114,256,392 on chromosome 2, position 28,829,182 on chromosome 6, position 144,601,781 on chromosome 8, position 144,601,800 on chromosome 8, and position 35,700,382 on chromosome 6, and the CpG sites located in the vicinity thereof.

Thus, the present invention enables early detection of NASH patients with high risk of developing hepatocellular carcinoma. The present invention provides incentives to seek follow-up consultations to NASH patients of the high-risk group of hepatocarcinogenesis. In addition, if the NASH patients with high risk of developing hepatocellular carcinoma can be discovered early by the present invention, preventive intervention can be performed to prevent the development of carcinoma, or early diagnosis or treatment of the carcinoma can improve their life prognosis.

Therefore, the present invention also relates to the preventive intervention in subjects having a risk of developing hepatocellular carcinoma detected by the method of the present invention. For example, the subjects having a risk of developing hepatocellular carcinoma can be given regular checkups (diagnostic imaging, liver biopsy, etc.), lifestyle guidance, diet therapy, exercise therapy, and the like. This allows the prevention, early diagnosis, or early treatment of hepatocellular carcinoma in NASH patient with the high-risk group.

Examples

Hereafter, the present invention is described in detail with examples, but the present invention is not limited to the following examples.

[Example 1]

The first step in predicting future development of hepatocellular carcinoma from NASH is to distinguish liver tissue presenting with histological findings of NASH, which is the origin of hepatocellular carcinoma development (NASH associated with hepatocellular carcinoma [NASH-W]) from normal liver tissue [NLT]. Therefore, a genome-wide DNA methylation analysis was first performed using the high-density bead array Infinium™ Human Methylation 450 BeadChip.

The Infinium™ Human Methylation 450 BeadChip contains probes for methylation detection, each targeting a different CpG site. The DNAs prepared from 22 NASH-W specimens and 36 NLT specimens were treated with bisulfite and hybridized to the probes on the chip, then fluorescently-labeled bases were incorporated by single-base extension reaction, and the fluorescence signal from the incorporated labels was measured to detect methylation of the CpG sites targeted by the probes. 4,050 probes were identified in which the DNA methylation rate was significantly different between the 22 NASH-W specimens and the 36 NLT specimens, and the difference between the average values was also sufficiently great (Welch-T Test, Bonferroni correction, $\Delta\beta>0.1$). When a receiver operating characteristic [ROC] curve analysis to distinguish NASH-W from NLT was performed using these 4,050 probes, there were 437 probes with an area under the curve [AUC] greater than 0.95, i.e., with high discriminative power (Figure 1).

However, considering the clinical practice, it is not realistic to compare NASH-W with NLT. In reality, the risk of hepatocarcinogenesis is presumably predicted after a diagnosis of NASH is confirmed by liver biopsy, in order to determine the course of treatment and follow-up. That is, it is important to compare liver tissue presenting with histological findings of NASH but without hepatocellular carcinoma [NASH-O] and NASH-W associated with hepatocellular carcinoma. On the other hand, cases currently analyzed as NASH-O may develop hepatocellular carcinoma in the future if preventive interventions or the like are not implemented. That is, a diagnostic indicator distinguishing NASH-W from NASH-O with 100% sensitivity and specificity is inappropriate.

Therefore, thresholds were set using the Yoden method for all 437 probes with AUCs greater than 0.95 which distinguish NASH-W from NLT, to create tentative predictive criteria. Using these predictive criteria, and considering the probability of developing hepatocellular carcinoma (carcinogenic rate) from NASH, which is said to be 11% from the cirrhosis stage, as well as to be on the safe side since this is a risk prediction for follow-up, the "probes with which less than 15% of the 91 NASH-O specimens having not yet associated with hepatocellular carcinoma are assessed as having a carcinogenic risk" were extracted. Of the 437 probes, there were 24 probes which detected less than 15% of the 91 NASH-O specimens based on the predictive criteria (Table 3, Figure 2). The 24 CpG sites listed in Table 3 which are detected by these 24 probes are marker CpG sites for predicting the carcinogenic risk from NASH, and predicting the carcinogenic risk from NASH based on the DNA methylation levels of these marker CpG sites is considered as "the method for predicting the carcinogenic risk from NASH developed in the present invention".

[Table 3]

| Probe ID of Infinium™ Human Methylation 450 BeadChip | Marker CpG Detected by Probe | | Area under the curve (AUC) to distinguish NASH-W already associated with hepatocellular carcinoma from normal liver tissue NLT | Threshold by Youden method | Test for difference in means between NASH-W and NLT (Welch T) | FDR of test for difference in means between NASH-W and NLT (Welch T) | DNA Methylation Status |
|---|---|---|---|---|---|---|---|
| | Chromosome Number | Position on the Chromosome (Build 37) | | | | | |
| cg09580822 | 10 | 130834003 | 0.973484848 | 0.68246825 | 4.24E-09 | 2.45E-07 | Reduced |
| cg14950303 | 2 | 114256392 | 0.957142857 | 0.5189492 | 6.08E-12 | 2.09E-09 | Increased |
| cg15050398 | 6 | 28829182 | 0.958333333 | 0.47324505 | 3.54E-13 | 2.88E-10 | Reduced |
| cg18210511 | 8 | 144601781 | 0.955808081 | 0.52068635 | 2.53E-11 | 5.71E-09 | Increased |
| cg09580859 | 8 | 144601800 | 0.950757576 | 0.6527342 | 9.40E-11 | 1.47E-08 | Increased |
| cg13719443 | 6 | 35700382 | 0.951948052 | 0.41313305 | 1.92E-13 | 1.95E-10 | Reduced |
| cg00431813 | 7 | 1051703 | 0.95959596 | 0.54246655 | 2.91E-12 | 1.23E-09 | Increased |
| cg05393736 | 10 | 64892616 | 0.95959596 | 0.70110665 | 3.02E-12 | 1.26E-09 | Increased |
| cg11820154 | 8 | 29732714 | 0.953282828 | 0.51279625 | 3.60E-11 | 7.42E-09 | Increased |
| cg13073141 | 1 | 150948024 | 0.953282828 | 0.5416704 | 4.42E-13 | 3.42E-10 | Increased |
| cg23785719 | 7 | 43622659 | 0.962121212 | 0.26819255 | 1.87E-13 | 1.91E-10 | Increased |
| cg25045113 | 17 | 8702099 | 0.950649351 | 0.68551085 | 2.10E-12 | 1.02E-09 | Increased |
| cg26583598 | 5 | 60776693 | 0.953282828 | 0.7271926 | 1.68E-12 | 8.59E-10 | Increased |
| cg05861743 | 6 | 31624387 | 0.950757576 | 0.8079586 | 3.46E-13 | 2.83E-10 | Increased |
| cg14288091 | 8 | 99305661 | 0.951948052 | 0.18628835 | 1.54E-11 | 4.04E-09 | Reduced |
| cg26385097 | 2 | 438095 | 0.953282828 | 0.3664063 | 8.92E-12 | 2.75E-09 | Reduced |
| cg22083325 | 17 | 66194721 | 0.981818182 | 0.2453593 | 5.52E-10 | 5.37E-08 | Increased |
| cg19182986 | 11 | 124613500 | 0.960858586 | 0.5857264 | 2.75E-11 | 6.10E-09 | Increased |
| cg26894854 | 1 | 205631084 | 0.968434343 | 0.4934916 | 1.15E-09 | 9.22E-08 | Increased |
| cg04662828 | 2 | 133039083 | 0.963383838 | 0.5250343 | 3.93E-12 | 1.53E-09 | Reduced |
| cg11152384 | 11 | 68934300 | 0.957070707 | 0.4285967 | 2.92E-12 | 1.23E-09 | Reduced |

| Probe ID of Infinium™ Human Methylation 450 BeadChip | Marker CpG Detected by Probe | | Area under the curve (AUC) to distinguish NASH-W already associated with hepatocellular carcinoma from normal liver tissue NLT | Threshold by Youden method | Test for difference in means between NASH-W and NLT (Welch T) | FDR of test for difference in means between NASH-W and NLT (Welch T) | DNA Methylation Status |
|---|---|---|---|---|---|---|---|
| | Chromos ome Number | Position on the Chromosome (Build 37) | | | | | |
| cg08912801 | 1 | 3399260 | 0.973484848 | 0.6030588 | 5.15E-11 | 9.52E-09 | Reduced |
| cg16319310 | 10 | 52390957 | 0.987373737 | 0.6061517 | 1.81E-16 | 2.37E-12 | Reduced |
| cg19861632 | 4 | 22392700 | 0.963383838 | 0.54526785 | 8.15E-09 | 4.02E-07 | Increased |

[Example 2]

A multivariate analysis was performed to identify the 22 NASH-W specimens associated with hepatocellular carcinoma from a total of 113 specimens, 91 NASH-O specimens and 22 NASH-W specimens. All 24 marker CpG sites listed in Table 3 were significant in the multivariate analysis. In the same multivariate analysis, no significance was observed for the histopathological findings. This indicates that the DNA methylation levels of each of the marker CpG sites listed in Table 3 can be used to predict the carcinogenic risk from NASH independently of histopathological findings and the like (Figure 3).

[Example 3]

To get an overview of the DNA methylation profile of NASH specimens, diagnostic criteria using the DNA methylation levels of the marker CpG sites listed in Table 3 were applied to the 91 NASH-O specimens and 22 NASH-W specimens. The results showed that NASH-W tended to meet the diagnostic criteria for methylation level of more CpG sites compared to NASH-O, while a group of cases showed a DNA methylation status similar to NASH-W which has already been associated with hepatocellular carcinoma at the NASH-O stage which has not yet been associated with hepatocellular carcinoma (Figure 4). This group of cases may be a group of cases with high risk of developing hepatocellular carcinoma and requiring close follow-up, which supports that the methylation level of the marker CpG sites in Table 3 reflects the risk of developing hepatocellular carcinoma.

[Example 4]

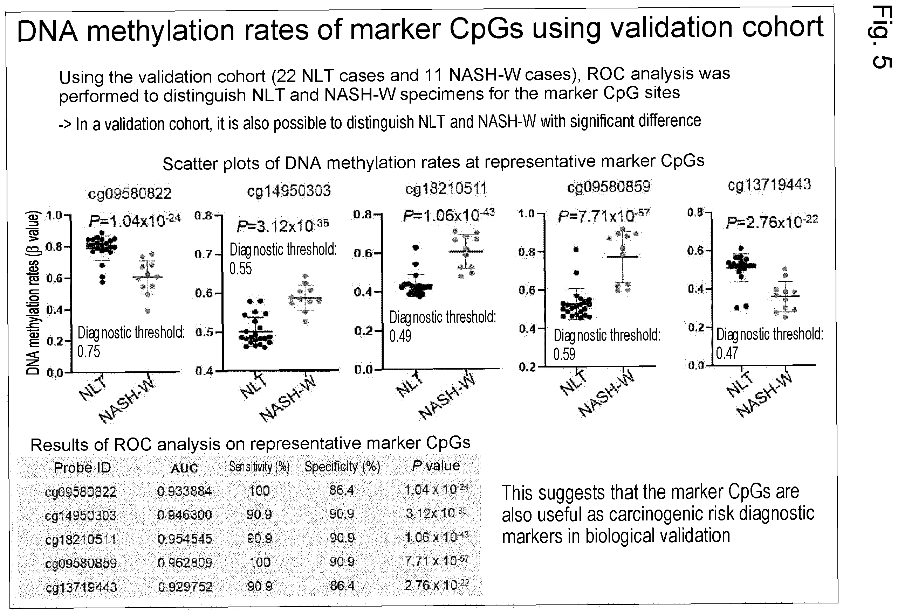

Using a validation cohort (22 NLT specimens and 11 NASH-W specimens), a ROC analysis was performed to distinguish NLT and NASH-W specimens for five of the marker CpG sites listed in Table 3. For all the CpG sites investigated, it was possible to distinguish NLT and NASH-W specimens with an AUC>0.92, a sensitivity >90.9%, and a specificity >86.4% (Figure 5). These results clearly show that the CpG sites listed in Table 3 are also useful as carcinogenic risk diagnostic markers in biological validation.

[Example 5]

NASH-W and NLT specimens were discriminated using methylation analysis by high-performance liquid chromatography (HPLC) (see WO 2014/136930). The marker CpG sites of Table 3 were used as the target CpG sites. The DNA prepared from the specimens was treated with bisulfite and the regions containing the target CpG sites were amplified by PCR. The amplification products were purified and subjected to HPLC. As references, a methylated control (target region DNA with 100% methylated CpG sites) and an unmethylated control (target region DNA with 0% methylated CpG sites) were similarly treated and subjected to HPLC. The methylation rate of specimen-derived DNA was determined from the calibration curve prepared based on the methylated and unmethylated controls. The column used for HPLC and the HPLC conditions are described below.

(Preparation of anion exchange column)

To 2000 mL of 3 wt% polyvinyl alcohol (manufactured by Nippon Synthetic Chemical Industry Co., Ltd.) aqueous solution in a reactor with a stirrer was added a mixture of 200 g of tetraethylene glycol dimethacrylate (manufactured by Shin-Nakamura Chemical Co., Ltd.), 100 g of triethylene glycol dimethacrylate (manufactured by Shin-Nakamura Chemical Co., Ltd.), 100 g of glycidyl methacrylate (manufactured by Wako Pure Chemical Industries, Ltd.) and 1.0 g of benzoyl peroxide (manufactured by Kishida Chemical Co., Ltd.). The mixture was heated while stirring, and polymerized at 80°C for 1 hour under a nitrogen atmosphere. Next, as a hydrophilic monomer having a strong cationic group, 100 g of trimethylammonium ethyl methacrylate chloride (manufactured by Wako Pure Chemical Industries, Ltd.) was dissolved in ion-exchanged water. This was added to the same reactor and was similarly polymerized at 80°C for 2 hours under a nitrogen atmosphere while stirring. The obtained polymerized composition was washed with water and acetone to obtain coated polymer particles having, on their surface, a layer of hydrophilic polymer having quaternary ammonium groups. When the obtained coated polymer particles were measured using a particle size distribution analyzer (AccuSizer 780 manufactured by Particle Sizing Systems), the average particle diameter was 10 μm. 10 g of the obtained coated polymer particles were dispersed in 100 mL of ion-exchanged water to prepare a pre-reaction slurry. Next, 10 mL of N,N-dimethylaminopropylamine (manufactured by Wako Pure Chemical Industries, Ltd.), a reagent having a weak cationic group, was added to this slurry while stirring, and the mixture was reacted at 70°C for 4 hours. After the reaction, the supernatant was removed using a centrifuge ("Himac CR20G" manufactured by Hitachi, Ltd.) and washed with ion-exchanged water. After washing, the supernatant was removed using a centrifuge. This washing with ion-exchanged water was repeated four more times to obtain a packing material for ion exchange chromatography having quaternary ammonium groups and tertiary amino groups on the surface of the base material particles. The obtained packing material for ion exchange chromatography was packed into a stainless steel column (column size: inner diameter 4.6 mm × length 150 mm) of a liquid chromatography system.

(HPLC conditions)

System: LC-20A series (manufactured by Shimadzu Corporation)
Column: anion exchange column (as prepared above)
Eluent: Eluent A 25mM MES-NaOH (pH6.0)
Eluent B 25mM MES-NaOH (pH6.0)
+ 2M Guanidine sulfate
Analysis time: 15 min
Elution method: the mixing ratio of eluent B was linearly increased according to the following gradient conditions:
0 min (30% eluent B) → 10 min (50% eluent B)
Flow rate: 1.0 mL/min
Detection wavelength: 260 nm
Sample injection amount: 5 μL
Column temperature: 70°C

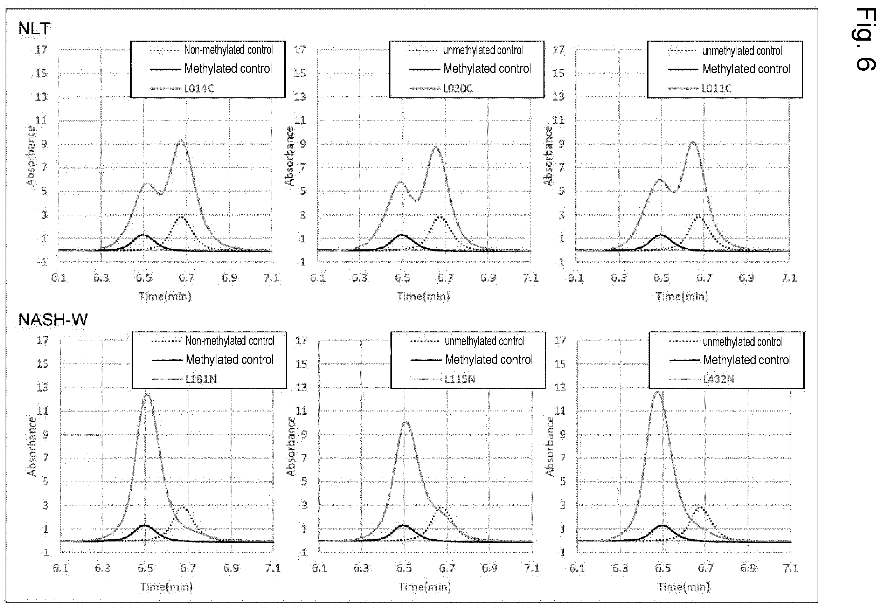

Examples of peak patterns detected from 3 NASH-W specimens and 3 NLT specimens for one CpG site are shown in Figure 6. The CpG site shown in Figure 6 (probe ID: cg18210511) is a site at which methylation is shown in the high-risk group and the HPLC peak patterns showed an increased DNA methylation level in the NASH-W specimens compared to the NLT specimens.

[Example 6]

For the marker CpG sites listed in Table 3, the following items 1) to 6) were evaluated and the CpG sites which exceeded the standard values were extracted. The CpG sites which met four or more of the six standards were extracted.

1) CpG Ratio

The content of CpG sequences in the sequences to be amplified in the PCR performed as part of the HPLC method in Example 5 was calculated. For example, if the sequence to be amplified by PCR is 276 bp and the number of CpG sequences contained in the sequence is 10, $100 \times (10 \div 276) = 3.62\%$. The standard value was set at 5%.

2) Degree of Control Separation

For each marker CpG site in the clinical specimens (30 NLT specimens and 20 NASH-W specimens, for a total of 50 specimens), the elution times and peak widths at half height for the methylated control peak and unmethylated control peak obtained by HPLC method according to the procedure in Example 5 were detected. These were applied to the following equation to calculate the degree of separation. The standard value was set at 0.8.

$$\text{Degree of Separation} = 1.18 \times (\text{elution time of unmethylated control peak} - \text{elution time of methylated control peak}) / (\text{peak width at half height of unmethylated control} + \text{peak width at half height of methylated control})$$

3) Correlation of Methylation Rate

For each marker CpG site in the same clinical specimen, the correlation between the methylation rate determined by HPLC method according to the procedure in Example 5 and the methylation rate analyzed by the Infinium™ method was confirmed, and the correlation coefficient ($R^2$) was calculated. The standard value was set at $R^2 > 0.45$.

4) Concordance Rate of Assessment

In a same clinical specimen, the methylation rate of each marker CpG site determined by HPLC method according to the procedure in Example 5 was used to assess NLT/NASH-W based on the cutoff values, and the concordance rate of the assessment with the clinical information was calculated. The standard value was set at 80%.

$$\text{Concordance rate of assessment} = 100 \times (\text{number of NLT assessed as NLT} + \text{number of NASH-W assessed as NASH-W}) / \text{total number of specimens evaluated}$$

5) Sensitivity

In a same clinical specimen, the methylation rate of each marker CpG site determined by HPLC method according to the procedure in Example 5 was used to assess NLT/NASH-W based on the cutoff values, and the percentage of NASH-W specimens assessed as NASH-W (sensitivity) was calculated. The standard value was set at 80%.

$$\text{Sensitivity} = 100 \times \text{number of NASH-W specimens assessed as NASH-W} / \text{NASH-W specimens}$$

6) Specificity

In a same clinical specimen, the methylation rate of each marker CpG site determined by HPLC method according to the procedure in Example 5 was used to assess NLT/NASH-W with cutoff values, and the percentage of NLT specimens assessed as NLT (specificity) was calculated. The standard value was set at 80%.

$$\text{Specificity} = 100 \times \text{number of NLT specimens assessed as NLT} / \text{NLT specimens}$$

Six of the 24 CpG sites listed in Table 3 (probe IDs: cg09580822, cg14950303, cg15050398, cg18210511, cg09580859 and cg13719443, see Table 3) met four or more of the above criteria 1) to 6). These six CpG sites were found to be preferable markers for predicting the carcinogenic risk from NASH. These six CpG sites are also useful as markers for predicting the carcinogenic risk using methylation analysis by HPLC method for the following reasons: presence of a relatively high number of CpG sites in the vicinity; high degree of separation between the peaks of the methylated and unmethylated controls in ion exchange chromatography analysis; or strong association between methylation level and development of hepatocellular carcinoma.

The results of the above examples show that the CpG sites listed in Table 3 are very useful as indicators for predicting the risk of developing hepatocellular carcinoma from NASH. The CpG sites listed in Table 3 are believed to be sufficiently applicable in clinical testing as markers for assessing the risk of developing hepatocellular carcinoma from NASH, for the realization of preemptive and personalized medicine from an epigenomic perspective.

**Claims**

1. A method for detecting hepatocytes or tissue comprising hepatocytes having a risk of developing hepatocellular carcinoma, the method comprising:

detecting DNA methylation level of a target CpG site in genomic DNA derived from hepatocytes or tissue comprising hepatocytes with non-alcoholic steatohepatitis; and

detecting hepatocytes or tissue comprising hepatocytes having a risk of developing hepatocellular carcinoma, from the detected DNA methylation level,

wherein the target CpG site is at least one CpG site selected from the group consisting of the CpG sites located at or in the vicinity of the positions on the chromosome described in Table 1.

[Table 1]

| Target CpG Site | |
|---|---|
| Chromosome Number | Position on the Chromosome |
| 10 | 130834003 |
| 2 | 114256392 |
| 6 | 28829182 |
| 8 | 144601781 |
| 8 | 144601800 |
| 6 | 35700382 |
| 7 | 1051703 |
| 10 | 64892616 |
| 8 | 29732714 |
| 1 | 150948024 |
| 7 | 43622659 |
| 17 | 8702099 |
| 5 | 60776693 |
| 6 | 31624387 |
| 8 | 99305661 |
| 2 | 438095 |
| 17 | 66194721 |
| 11 | 124613500 |
| 1 | 205631084 |
| 2 | 133039083 |
| 11 | 68934300 |
| 1 | 3399260 |
| 10 | 52390957 |
| 4 | 22392700 |

2. A method for detecting a subject having a risk of developing hepatocellular carcinoma, the method comprising the following:

detecting DNA methylation level of a target CpG site in genomic DNA derived from hepatocytes or tissue comprising hepatocytes of a subject with non-alcoholic steatohepatitis; and

detecting a subject having a risk of developing hepatocellular carcinoma from the detected DNA methylation level, wherein the target CpG site is at least one CpG site selected from the group consisting of the CpG sites located at or in the vicinity of the positions on the chromosome described in Table 2.

[Table 2]

| Target CpG Site | |
| --- | --- |
| Chromosome Number | Position on the Chromosome |
| 10 | 130834003 |
| 2 | 114256392 |
| 6 | 28829182 |
| 8 | 144601781 |
| 8 | 144601800 |
| 6 | 35700382 |
| 7 | 1051703 |
| 10 | 64892616 |
| 8 | 29732714 |
| 1 | 150948024 |
| 7 | 43622659 |
| 17 | 8702099 |
| 5 | 60776693 |
| 6 | 31624387 |
| 8 | 99305661 |
| 2 | 438095 |
| 17 | 66194721 |
| 11 | 124613500 |
| 1 | 205631084 |
| 2 | 133039083 |
| 11 | 68934300 |
| 1 | 3399260 |
| 10 | 52390957 |
| 4 | 22392700 |

**3.** A method for acquiring data to detect hepatocytes or tissue comprising hepatocytes having a risk of developing hepatocellular carcinoma, or a subject having a risk of developing hepatocellular carcinoma, the method comprising the following:

detecting DNA methylation level of a target CpG site in genomic DNA derived from hepatocytes or tissue comprising hepatocytes of a subject with non-alcoholic steatohepatitis; and
acquiring data on whether the hepatocytes, the tissue comprising hepatocytes, or the subject have a risk of developing hepatocellular carcinoma, from the detected DNA methylation level,
wherein the target CpG site is at least one CpG site selected from the group consisting of the CpG sites located at or in the vicinity of the positions on the chromosome described in Table 3.

[Table 3]

| Target CpG Site | |
|---|---|
| Chromosome Number | Position on the Chromosome |
| 10 | 130834003 |
| 2 | 114256392 |
| 6 | 28829182 |
| 8 | 144601781 |
| 8 | 144601800 |
| 6 | 35700382 |
| 7 | 1051703 |
| 10 | 64892616 |
| 8 | 29732714 |
| 1 | 150948024 |
| 7 | 43622659 |
| 17 | 8702099 |
| 5 | 60776693 |
| 6 | 31624387 |
| 8 | 99305661 |
| 2 | 438095 |
| 17 | 66194721 |
| 11 | 124613500 |
| 1 | 205631084 |
| 2 | 133039083 |
| 11 | 68934300 |
| 1 | 3399260 |
| 10 | 52390957 |
| 4 | 22392700 |

4. The method according to any one of claims 1 to 3, wherein the target CpG site is at least one CpG site selected from the group consisting of the CpG sites at position 130,834,003 on chromosome 10, position 114,256,392 on chromosome 2, position 28,829,182 on chromosome 6, position 144,601,781 on chromosome 8, position 144,601,800 on chromosome 8, and position 35,700,382 on chromosome 6, and the CpG sites located in the vicinity thereof.

5. The method according to any one of claims 1 to 4, wherein the detection of the DNA methylation level comprises detecting the DNA methylation level of the target CpG site using the genomic DNA treated with bisulfite.

Fig. 1

EP 4 074 841 A1

# Diagnosis of the risk of developing hepatocellular carcinoma from NASH is an unmet medical need

The first step in predicting future development of hepatocellular carcinoma is to distinguish NASH-W associated with hepatocellular carcinoma from normal liver tissue NLT

Significant difference between normal liver tissue NLT (n=36) vs NASH-W associated with hepatocellular carcinoma (n=22), and difference is sufficient
= 4,050 probes (Welch-T Test, Bonferroni correction, $\Delta\beta>0.1$) -> DNA methylation abnormalities are definitively occurring in NASH

Receiver operating characteristic (ROC) analysis to distinguish NASH-W from NLT $\Rightarrow$ Area under the curve (AUC) is greater than 0.95 = a high number of probes (437) with high discriminative power

ROC curve: 0.665 (0.909, 0.934)

Axes: Sensitivity (y), Specificity (x)

| Probe ID | AUC | $\Delta\beta_{NASH-NLT}$ | Probe ID | AUC | $\Delta\beta_{NASH-NLT}$ |
|---|---|---|---|---|---|
| cg18457818 | 0.994 | 0.144 | cg07959490 | 0.981 | 0.159 |
| cg19349861 | 0.992 | 0.100 | cg13161852 | 0.981 | -0.103 |
| cg07724654 | 0.990 | 0.115 | cg13468249 | 0.981 | 0.124 |
| cg14427382 | 0.990 | -0.138 | cg20251199 | 0.981 | 0.124 |
| cg21980338 | 0.990 | 0.121 | cg15284457 | 0.976 | 0.782 |
| cg24147392 | 0.990 | 0.133 | cg15646741 | 0.976 | 0.457 |
| cg03505117 | 0.987 | 0.103 | cg15920791 | 0.976 | 0.313 |
| cg15606745 | 0.987 | -0.110 | cg18159180 | 0.976 | 0.434 |
| cg16319310 | 0.987 | -0.111 | cg20326359 | 0.976 | 0.823 |
| cg03571507 | 0.986 | -0.130 | cg22216431 | 0.976 | 0.505 |
| cg12424965 | 0.986 | 0.125 | cg22793065 | 0.976 | 0.325 |
| cg14381908 | 0.986 | 0.168 | cg02073839 | 0.975 | 0.749 |
| cg25824760 | 0.986 | 0.140 | cg03468541 | 0.975 | 0.164 |
| cg20426671 | 0.985 | 0.127 | cg04449562 | 0.975 | 0.730 |
| cg14299696 | 0.984 | 0.122 | cg04867257 | 0.975 | 0.493 |
| cg12074375 | 0.984 | -0.128 | cg05707844 | 0.975 | 0.250 |
| cg21152376 | 0.984 | 0.105 | cg08941173 | 0.975 | 0.356 |
| cg25987208 | 0.983 | 0.129 | cg15936935 | 0.975 | 0.611 |
| cg07076175 | 0.982 | 0.103 | cg19105961 | 0.975 | 0.332 |
| cg07738077 | 0.982 | -0.153 | cg15470736 | 0.974 | 0.788 |
| cg07886142 | 0.982 | -0.114 | cg00112042 | 0.973 | 0.192 |
| cg11070069 | 0.982 | 0.142 | cg01593421 | 0.973 | 0.574 |
| cg11213199 | 0.982 | 0.105 | cg03000596 | 0.973 | 0.630 |
| cg12809925 | 0.982 | 0.182 | cg03022552 | 0.973 | 0.781 |
| cg22083325 | 0.982 | 0.109 | cg08912801 | 0.973 | 0.603 |
| cg07138452 | 0.981 | 0.124 | | | |
| cg07956857 | 0.981 | -0.132 | | | |

Fig. 2

**Identification of candidate CpG sites for predicting carcinogenic risk**

0.665 (0.909, 0.934)

Yoden method:
maximization of distance

Sensitivity / Specificity

Tentative prediction criteria were created by setting diagnostic thresholds for the 437 probes with AUCs greater than 0.95 which distinguish NASH-W from NLT

Considering of the carcinogenic rate from NASH, "probes with which less than 15% of the NASH-O which have not yet associated with hepatocellular carcinoma are assessed as having a carcinogenic risk" were extracted

= 24 probes

# Comparison between prediction panel of risk of hepatocarcinogenesis and histological findings

It is desirable to be able to predict by the method of the present invention the carcinogenic risk which cannot be predicted by the histopathological findings of biopsy specimens alone

## [Multivariate analysis for predicting carcinogenic risk]

Distinguish 22 NASH-W specimens associated with hepatocellular carcinoma from the 91 NASH-O specimens without hepatocellular carcinoma and the 22 NASH-W specimens associated with hepatocellular carcinoma (total: 113 specimens)

cg00431813

| Variable | Odds Rate | 95% Confidence Interval | P |
|---|---|---|---|
| Ballooning | 5.7861 | 0.1324-252.8274 | 0.362 |
| NASH stage (Brunt classification) | 0.6860 | 0.2746-1.7136 | 0.419 |
| DNA methylation diagnosis | 232.0588 | 31.1654-1727.9146 | < 0.001 |

cg09580822

| Variable | Odds Rate | 95% Confidence Interval | P |
|---|---|---|---|
| Ballooning | 1.3780 | 0.1234-15.3824 | 0.794 |
| NASH stage (Brunt classification) | 0.5532 | 0.2479-1.2345 | 0.148 |
| DNA methylation diagnosis | 180.6101 | 23.7570-1361.6031 | < 0.001 |

cg05393736

| Variable | Odds Rate | 95% Confidence Interval | P |
|---|---|---|---|
| Ballooning | 4.2859 | 0.2687-68.3402 | 0.302 |
| NASH stage (Brunt classification) | 0.9718 | 0.4720-2.0005 | 0.938 |
| DNA methylation diagnosis | 44.9767 | 10.9341-185.0085 | < 0.001 |

cg11820154

| Variable | Odds Rate | 95% Confidence Interval | P |
|---|---|---|---|
| Ballooning | 0.4597 | 0.0312-6.7648 | 0.571 |
| NASH stage (Brunt classification) | 0.3471 | 0.0998-1.2069 | 0.096 |
| DNA methylation diagnosis | 1138.4922 | 52.7399-24576.5205 | < 0.001 |

All of the diagnostic criteria using the DNA methylation rates of the 24 marker CpG sites suggested the possibility of diagnosing carcinogenic risk independently of histopathological findings

Fig. 3

Fig. 4

EP 4 074 841 A1

# DNA methylation profiles of NASH specimens

To get an overview of the DNA methylation profile of NASH specimens, diagnostic criteria using the DNA methylation rates of the marker CpG sites were applied to the 91 NASH-O specimens and 22 NASH-W specimens (total: 113 specimens)

NASH-O which have been not yet associated hepatocellular carcinoma

NASH-W which have already developed hepatocellular carcinoma (n=22)

NASH-O shows bimodality with clear valleys

Number of cases

Number of CpG sites meeting the diagnostic criteria (how many diagnostic criteria were positive)

Second peak overlaps with NASH-W which have already developed hepatocellular carcinoma
= Group of cases requiring close follow-up as having a high risk of developing hepatocellular carcinoma?

# Fig. 5

## DNA methylation rates of marker CpGs using validation cohort

Using the validation cohort (22 NLT cases and 11 NASH-W cases), ROC analysis was performed to distinguish NLT and NASH-W specimens for the marker CpG sites

-> In a validation cohort, it is also possible to distinguish NLT and NASH-W with significant difference

### Scatter plots of DNA methylation rates at representative marker CpGs

cg09580822 — $P=1.04 \times 10^{-24}$ — Diagnostic threshold: 0.75

cg14950303 — $P=3.12 \times 10^{-35}$ — Diagnostic threshold: 0.55

cg18210511 — $P=1.06 \times 10^{-43}$ — Diagnostic threshold: 0.49

cg09580859 — $P=7.71 \times 10^{-57}$ — Diagnostic threshold: 0.59

cg13719443 — $P=2.76 \times 10^{-22}$ — Diagnostic threshold: 0.47

DNA methylation rates (β value); NLT; NASH-W

### Results of ROC analysis on representative marker CpGs

| Probe ID | AUC | Sensitivity (%) | Specificity (%) | P value |
|---|---|---|---|---|
| cg09580822 | 0.933884 | 100 | 86.4 | $1.04 \times 10^{-24}$ |
| cg14950303 | 0.946300 | 90.9 | 90.9 | $3.12 \times 10^{-35}$ |
| cg18210511 | 0.954545 | 90.9 | 90.9 | $1.06 \times 10^{-43}$ |
| cg09580859 | 0.962809 | 100 | 90.9 | $7.71 \times 10^{-57}$ |
| cg13719443 | 0.929752 | 90.9 | 86.4 | $2.76 \times 10^{-22}$ |

This suggests that the marker CpGs are also useful as carcinogenic risk diagnostic markers in biological validation

Fig. 6

## INTERNATIONAL SEARCH REPORT

| International application No. |
| --- |
| PCT/JP2020/045879 |

### A. CLASSIFICATION OF SUBJECT MATTER

C12Q 1/6869(2018.01)i
FI: C12Q1/6869 Z

According to International Patent Classification (IPC) or to both national classification and IPC

### B. FIELDS SEARCHED

Minimum documentation searched (classification system followed by classification symbols)
C12Q1/6869

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
CAplus/MEDLINE/EMBASE/BIOSIS/WPIDS (STN), JSTPlus/JMEDPlus/JST7580 (JDreamIII), PubMed, Japio-GPG/FX

### C. DOCUMENTS CONSIDERED TO BE RELEVANT

| Category* | Citation of document, with indication, where appropriate, of the relevant passages | Relevant to claim No. |
| --- | --- | --- |
| A | JIAYU, W. et al., "Altered DNA Methylation Sites in Peripheral Blood Leukocytes from Patients with Simple Steatosis and Nonalcoholic Steatohepatitis (NASH).", Medical Science Monitor, 2018, vol. 24, pp. 6946-6967, abstract, fig. 3, 5, table 2, page 6948, right column, paragraph [0003] | 1-5 |
| A | ZHANG, R. N. et al., "Genome-wide analysis of DNA methylation in human peripheral leukocytes identifies potential biomarkers of nonalcoholic fatty liver disease.", International Journal of Molecular Medicine, 2018, vol. 42, pp. 443-452, abstract, tables 3, 4, fig. 4, 5, page 444, right column, paragraph [0002] | 1-5 |

☒ Further documents are listed in the continuation of Box C.    ☒ See patent family annex.

| | |
| --- | --- |
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 04 February 2021 (04.02.2021) | 22 February 2021 (22.02.2021) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2020/045879 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | DE MELLO, V. D. et al., "Human liver epigenetic alterations in non-alcoholic steatohepatitis are related to insulin action.", EPIGENETICS, 2017, vol. 12, no. 4, pp. 287-295, abstract, fig. 1, table 2 | 1-5 |
| A | KURAMOTO, J. et al., "Genome-wide DNA methylation analysis during non-alcoholic steatohepatitis-related multistage hepatocarcinogenesis: comparison with hepatitis virus-related carcinogenesis.", Carcinogenesis, 2017, vol. 38, no. 3, pp. 261-270, abstract, fig. 3-5 | 1-5 |
| A | US 2018/0135130 A1 (VIRGINIA COMMONWEALTH UNIVERSITY) 17 May 2018 (2018-05-17) claim 1, table 2 | 1-5 |

Form PCT/ISA/210 (continuation of second sheet) (January 2015)

**INTERNATIONAL SEARCH REPORT**

Information on patent family members

International application No.

PCT/JP2020/045879

| Patent Documents referred in the Report | Publication Date | Patent Family | Publication Date |
|---|---|---|---|
| US 2018/0135130 A1 | 17 May 2018 | WO 2016/187030 A1<br>claim 1, table 2<br>EP 3294748 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013168644 A
- WO 2015129916 A
- WO 2017038983 A
- WO 2020116573 A
- WO 2019181941 A
- WO 2019039532 A
- WO 2014136930 A
- WO 2012108516 A

**Non-patent literature cited in the description**

- *Int J Oncol,* 2013, vol. 43, 1333-1342
- *Cancer sci,* 2010, vol. 101 (1), 36-45
- *Jpn J Can Res,* 1996, vol. 87 (12), 1210-1217
- *Int J Cancer,* 2009, vol. 125 (12), 2854-2862
- *Front Genet,* 2014, vol. 5, 24
- *Carcinogenesis,* 2017, vol. 38, 261-270
- *J Cancer Res Clin Oncol,* 2020, vol. 146 (10), 2461-2477
- *Nucleic Acids Res,* 1994, vol. 22, 2990-7
- *Anal Biochem,* 2000, vol. 10, 103-110
- *Nat Protoc,* 2008, vol. 3, 1903-8
- *Mutat Res,* 2005, vol. 573, 83-95